(19) Europäisches Patentamt / European Patent Office / Office européen des brevets

(11) **EP 4 548 908 A1**

(12) **EUROPEAN PATENT APPLICATION**

(43) Date of publication:
07.05.2025 Bulletin 2025/19

(21) Application number: 24209423.3

(22) Date of filing: 29.10.2024

(51) International Patent Classification (IPC):
**A61K 8/19** (2006.01)     **A61K 8/37** (2006.01)
**A61K 8/41** (2006.01)     **A61K 8/44** (2006.01)
**A61Q 5/10** (2006.01)     **A61Q 5/00** (2006.01)

(52) Cooperative Patent Classification (CPC):
**A61K 8/19; A61K 8/37; A61K 8/41; A61K 8/415;
A61K 8/44; A61K 8/447; A61Q 5/10;** A61Q 5/002

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: 31.10.2023  IT 202300022923

(71) Applicant: **Beauty & Business SpA**
**20123 Milano (IT)**

(72) Inventors:
• **CONSOLI, Antonio**
  **24059 Urgnano (BG) (IT)**
• **BESOZZI, Monica Silvia**
  **PONTIROLO NUOVO (BG) (IT)**
• **ALDEGANI, Nicola**
  **VERDELLINO (BG) (IT)**
• **GREVALCUORE, Katiuscia**
  **BERGAMO (IT)**

(74) Representative: **Bianchetti & Minoja with
Trevisan & Cuonzo IPS SRL
Via Plinio, 63
20129 Milano (IT)**

(54) **PERMANENT OXIDATIVE HAIR-COLOURING COMPOSITIONS**

(57) Disclosed are permanent hair-colouring compositions comprising aminophenol oxidative dyes and an alkaliser, characterised by a combination of cysteine and azelaic acid esters. The compositions according to the invention, mixed with an activator, colour the hair permanently and reproducibly without the unpleasant colour changes caused by storage, thus ensuring a deep, glossy colour result, and preserving the natural elasticity and strength of the hair.

**EP 4 548 908 A1**

## Description

The subject of the present invention is permanent hair-colouring compositions comprising aminophenol oxidative dyes and a combination of cysteine and azelaic acid esters.

## PRIOR ART

The main hair-colouring methods are the semi-permanent system and the permanent oxidative system. The first involves the use of direct dyes which are deposited on the hair surface. With this system, the colour gradually fades every time the hair is shampooed, and eventually disappears within 10 washes.

In the permanent system, colour is created by the reaction of primary intermediates and couplers in the presence of an oxidant. The stability of the permanent dye to washing ranges from four to eight weeks. The oxidative system is based on the reaction of "primary intermediates" with couplers, both of which are practically colourless. In the presence of air or oxidising agents such as hydrogen peroxide, primary dyes, typically p-phenylenediamine (PPD), p-toluenediamine (PTD), 2-methoxymethyl-p-phenylenediamine (MBB) and p-aminophenol (PAP), react with couplers such as resorcinol, m-aminophenol and p-amino-o-cresol (PAOC).

M-aminophenol is one of the couplers most widely used in hair-colouring preparations because, in combination with PPD, PTD and MBB, it produces violet-brown colours that are particularly useful to create natural, golden and copper shades. M-aminophenols (MAPs), and aminophenols in general, are highly sensitive to oxidation, and tend to degrade during storage.

Degradation of MAPs in particular generates a loss of the violet tone found especially in the lightest shades (very light, lightest and platinum blonde) or in violet, irisé or ash toners.

US2016/0214925 describes the problem of instability of aromatic dyes such as nitro compounds (4-nitro-o-phenylenediamine, 2-nitro-p-phenylenediamine, HC blue n 2 and HC yellow n 4), alpha-naphthol and 1,5-dihydroxynaphthalene, and especially m-aminophenols (MAPs) such as m-aminophenol (MAP), 5-amino-o-cresol (PAOC) and 5-(2-hydroxyethylamino)-2-methylphenol (PAOX) which, being particularly sensitive to oxidation, are not easily stabilised with the common antioxidants and chelating agents.

The amount of antioxidants cannot be increased as required without giving rise to an adverse effect on the permanent oxidative dyeing result because the antioxidant prevents colour development, thus producing unsatisfactory results.

Various antioxidants, such as ascorbic acid, cysteine and thioglycolic acid, are in fact used also in colour removal, as described in WO2007072892 and JP2006288613.

US2016/0214925 describes the use of resorcinol and derivatives thereof as MAP stabilisers.

Said solution involves both a formulation problem, because resorcinol and derivatives thereof cannot be introduced into all shades as they affect the colour result, and a problem associated with regulatory and legislative aspects, due to possible interference by resorcinol with endocrine processes.

There is consequently a need to eliminate resorcinol from cosmetic formulations, or limit the amount they contain. Oxidative hair-colouring preparations without resorcinol have been recently described, for example, in US11517514 and WO2023/163903.

US11285091 describes the removal of m-aminophenol from formulations, but does not solve the problem of other m-aminophenol (MAP) dyes and other dyes which are particularly unstable to oxidation.

US4875902 explains that some dyes are particularly liable to instability, especially in highly aerated formulations such as liquids and gels, and emphasises the inefficacy of conventional antioxidants such as ascorbic acid, thioglycolic acid, lactic acid, cysteine and derivatives thereof. The addition of TBHQ (t-butyl hydroquinone) to stabilise dyes is proposed as a solution.

JP4995441 and US20090265865 describe the use of flavonoids or extracts containing them. In both cases, not only are the solutions expensive, but flavonoids and extracts thereof can destabilise emulsions or interfere with the colour (Recent Advancements in Natural Plant Colorants Used for Hair Dye Applications: A Review, Molecules. 2022 Nov; 27(22): 8062).

US7753966 proposes the use of thiol compounds such as limonene thiol, thiogeraniol, thiomenthol, thioglycerin, p-menthene-8-thiol and 8-mercaptomenthone.

Despite various attempts, the problem of stabilisation of aminophenols in general, and MAPs in particular, has still not been entirely solved, and the loss of violet tone is still evident, especially in toners and superlighteners (very light, lightest and platinum blonde). Loss of tone is already evident after a few weeks' storage.

There is consequently a need to stabilise aminophenols to prevent loss of tone during storage and preserve the result in terms of colour, depth and shine.

**DESCRIPTION OF THE INVENTION**

It has been found that the combination of cysteine with specific azelaic acid esters gives rise to effective stabilisation of MAPs, even in absence of resorcinol, without affecting the depth and brightness of the colour result.

The subject of the invention is therefore permanent oxidative hair-colouring compositions comprising aminophenol dyes, an alkalising agent, cysteine, and particular azelaic acid esters selected from diethylhexyl azelate and dioctyldodecyl azelate or a mixture thereof.

The cysteine, optionally as hydrochloride or salified, can be in D, L or racemic form.

Examples of m-aminophenols include m-aminophenol (MAP), 5-amino-o-cresol (PAOC) and 5-(2-hydroxyethylamino)-2-methylphenol (PAOX).

The concentration by weight of cysteine or its salts ranges between 0.3% and 1%, while the concentration by weight of azelaic acid esters ranges between 0.05% and 2%.

The combination of cysteine and azelaic acid esters preserves the natural elasticity and strength of the hair, which is often adversely affected by permanent hair-colouring preparations, especially when superlightening shades (very light, lightest and platinum blonde) are used. The compositions according to the invention, mixed with an activator, colour the hair permanently and reproducibly without the undesirable colour changes caused by storage, thus ensuring a deep, glossy colour result, and preserving the natural elasticity and strength of the hair.

Suitable activators include hydrogen peroxide, carbamide peroxide, perborates and persulphates or peracids. Hydrogen peroxide, in concentrations ranging from 0.1 to 50% by weight, is preferred.

The compositions according to the invention can optionally be in ready-to-use form, comprising two or more ingredients to be mixed before use.

The pH of the compositions can range from 3 to 11, preferably between 6.5 and 11.

The alkalising agent is selected, for example, from ammonia, monoethanolamine (MEA), 1-amino-2-propanol, 2-amino-2-methyl-propanol (AMP), 2-amino-2-methyl-1,3-propanediol, 2-amino-2-ethyl-1,3-propanediol and tris(hydroxymethyl)-aminomethane (tromethamine, Tris), sodium hydroxide, potassium hydroxide, urea, allantoin, arginine, tripotassium phosphate, sodium saccharine, triethanolamine (TEA), dimethylglucamine (DMG), calcium sodium phosphosilicate or combinations thereof. The alkalising agent can also be a basic amino acid, preferably selected from arginine, lysine, omithine and histidine, more preferably arginine or lysine. The amount of alkalising agent can range between 0.1 and 20% by weight, preferably between 0.2 and 10% by weight.

The compositions can also include a pH regulator selected, for example, from sodium hydroxide, potassium hydroxide, urea, allantoin, tripotassium phosphate, sodium saccharine, triethanolamine, lactic acid, citric acid and phosphoric acid or combinations thereof.

The preferred dyes are listed below according to the INCI nomenclature (2006/257/EC as amended - International Nomenclature of Cosmetic Ingredients):

p-phenylenediamine (PPD), p-toluenediamine, (PTD); 2-metoxymethyl-p-phenylenediamine (MBB), p-aminophenol (PAP); N-methyl-p-aminophenol (photorex); 1-hydroxyethyl-4,5-diamino pyrazole; 3,4-methylenedioxyaniline HCl (HMOC); 2-amino-3-hydroxypyridine; 2-methyl-5-hydroxyethylaminophenol, 1-Acetoxy-2-Methylnaphthalene, 5-Amino-4-Chloro-o-Cresol, 4-Amino-m-Cresol, 6-Amino-m-Cresol, 3-Amino-2,4-Dichlorophenol, 6-Amino-2,4-Dichloro-m-Cresol, 3-Amino-2,4-Dichlorophenol, 5-Amino-2,6-Dimethoxy-3-Hydroxypyridine, 5-Amino-2,6-Dimethoxy-3-Hydroxypyridine, 3-Amino-2,6-Dimethylphenol, 2-Amino-5-Ethylphenol, 5-Amino-4-Fluoro-2-Methylphenol Sulphate, 2-Amino-4-Hydroxyethylaminoanisole, 2-Amino-4-Hydroxyethylaminoanisole, 2-Amino-3 -Hydroxypyridine, 4-Amino-2-Hydroxytoluene, 2-Aminomethyl-p-Aminophenol, 4-Amino-2-Nitrodiphenylamine-2'-Carboxylic Acid, m-Aminophenol, o-Aminophenol, 1,3-Bis-(2,4-Diaminophenoxy)propane, 4,6-Bis(2-Hydroxyethoxy)-m-Phenylenediamine, 2,6-Bis(2-Hydroxyethoxy)-3,5-Pyridinediamine, N,N-Bis(2-Hydroxyethyl)-p-Phenylenediamine, 4-Chloro-2-Aminophenol, 2-Chloro-p-Phenylenediamine, 4-Chlororesorcinol, N-Cyclopentyl-m-Aminophenol, 3,4-Diaminobenzoic Acid, 4,5-Diamino-1-((4-Chlorophenyl)Methyl)-1H-Pyrazole-Sulphate, 2,3-Diaminodihydropyrazolo Pyrazolone Dimethosulphonate, 2,4-Diaminodiphenylamine, 4,4'-Diaminodiphenylamine, 2,4-Diamino-5-Methylphenetole, 2,4-Diamino-5-Methylphenoxyethanol, 4,5-Diamino-1-Methylpyrazole, 2,4-Diaminophenol, 2,4-Diaminophenoxyethanol, 2,6-Diaminopyridine, 2,6-Diamino-3-((Pyridin-3-yl)Azo)Pyridine, N,N-Diethyl-m-Aminophenol, N,N-Diethyl-p-Phenylenediamine, N,N-Diethyltoluene-2,5-Diamine, 2,6-Dihydroxy-3,4-Dimethylpyridine, 2,6-Dihydroxyethyl-aminotoluene, Dihydroxyindole, Dihydroxyindoline, 2,6-Dimethoxy-3,5-Pyridinediamine, m-Dimethylaminophenyl Urea, N,N-Dimethyl-p-Phenylenediamine, 2,6-Dimethyl-p-Phenylenediamine, N,N-Dimethyl 2,6-Pyridinediamine, 4-Ethoxy-m-Phenylenediamine, 3-Ethylamino-p-Cresol, 4-Fluoro-6-Methyl-m-Phenylenediamine, 1-Hexyl-4,5-Diamino Pyrazole Sulphate, Hydroquinone, Hydroxyanthraquinoneaminopropyl Methyl Morpholinium Methosulphate, Hydroxybenzomorpholine, Hydroxyethoxy Aminopyrazolopyridine, Hydroxyethylaminomethyl-p-Aminophenol, Hydroxyethyl-2,6-Dinitro-p-Anisidine, Hydroxyethyl-p-Phenylenediamine, 2-Hydroxyethyl Picramic Acid, 6-Hydroxyindole, Hydroxypropyl Bis(N-Hydroxyethyl-p-Phenylenediamine), Hydroxypropyl-p-Phenylenediamine, Hydroxypyridinone, Isatin, N-Isopropyl 4,5-Diamino Pyrazole, N-Methoxyethyl-p-Phenylenediamine, 6-Methoxy-2-methylamino-3-aminopyridine, 2-Methoxymethyl-p-Aminophenol, 2-Methoxy-p-Phenylenediamine, 6-Methoxy-2,3-Pyridinediamine, 4-Methoxytoluene-2,5-Diamine, 4-Methylbenzyl 4,5-Diamino Pyrazole, 2,2'-Methylenebis 4-Aminophenol, 3,4-Methylenedioxyaniline, 3,4-Methylenedioxyphenol, 2-Methyl-5-Hydroxyethylaminophenol, Methylimidazoliumpropyl p-Phenylenediamine, 2-Methyl-1-Naphthol, 2-Methylresorcinol, 1,5-Naphthalenediol, 1,7-Naphthalenediol, 2,3-Naphthalenediol, 2,7-Naphthalenediol, 1-Naphthol, 2-Naphthol, PEG-3 2,2'-Di-p-Phenylenediamine, p-Phenetidine, m-Phenylenediamine, Phenyl Methyl Pyrazolone, N-Phenyl-p-Phenylenediamine, Picramic Acid, Pyrocatechol, Pyrogallol, Sodium Picramate, Tetraaminopyrimidine, Tetrahydro-6-Nitroquinoxaline, Tetrahydropyranyl, Toluene-2,6-Diamine, Toluene-3,4-Diamine, 2,5,6-Triamino-4-Pyrimidinol, 1,2,4-Trihydroxybenzene. The oxidative dyes can be in the form of salts.

The compositions preferably do not contain resorcinol.

The compositions according to the invention can also contain direct dyes. Examples of direct dyes, defined according to the INCI nomenclature, comprise:

Acid green 25, Acid blue 74, Acid blue 3, Acid blue 9, Acid red 18, Acid red 184, Acid red 195, Acid red 27, Acid red 33, Acid red 35, Acid red 51, Acid red 73, Acid red 87, Acid red 92, Acid red 95, Acid violet 43, Acid violet 9, Acid yellow 23, Acid yellow 3, Acid yellow 36, Acid yellow 73, Acid orange 6, Acid orange 7, Acid green 1, Acid green 50, Acid Blue 1, Acid Blue 62, Acid Brown 13, Acid Orange 3, Acid Orange 24, Acid Red 14, Acid Red 35, Acid Red 52, Acid Yellow 1, Acid Black 1, Acid blue 80, 2-Amino-6-Chloro-4-Nitrophenol, 4-Amino-2-Nitrodiphenylamine-2'-Carboxylic Acid, 2-Amino-3-Nitrophenol, 2-Amino-4-Nitrophenol, 2-Amino-5-Nitrophenol, 4-Amino-2-Nitrophenol, 4-Amino-3-Nitrophenol, Basic Blue 3, Basic Blue 7, Basic Blue 9, Basic Blue 26, Basic Blue 47, Basic Blue 75, Basic Blue 99, Basic Blue 124, Basic Brown 4, Basic Brown 16, Basic Brown 17, Basic Green 1, Basic Green 4, Basic Orange 1, Basic Orange 2, Basic Orange 31, Basic Red 1, Basic Red 1:1, Basic Red 2, Basic Red 22, Basic Red 46, Basic Red 51, Basic Red 76, Basic Red 118, Basic Violet 1, Basic Violet 2, Basic Violet 3, Basic Violet 4, Basic Violet 10, Basic Violet 11:1, Basic Violet 14, Basic Violet 16, Basic Yellow 28, Basic Yellow 40, Basic Yellow 57, Basic Yellow 87, N,N'-Bis(2-Hydroxyethyl)-2-Nitro-p-Phenylenediamine, 2-Chloro-6-Ethylamino-4-Nitrophenol, 2-Chloro-5-Nitro-N-Hydroxyethyl p-Phenylenediamine, N,N'-Dimethyl-N-Hydroxyethyl-3-Nitro-p-Phenylenediamine, Direct Black 51, Direct Red 23, Direct Red 80, Direct Red 81, Direct Violet 48, Direct Yellow 12, Disperse Black 9, Disperse Blue 1, Disperse Blue 3, Disperse Blue 7, Disperse Blue 377, Disperse Brown 1, Disperse Orange 3, Disperse Red 11, Disperse Red 15, Disperse Red 17, Disperse Violet 1, Disperse Violet 4, Disperse Violet 15, HC Blue No. 2, HC Blue No. 4, HC Blue No. 5, HC Blue No. 6, HC Blue No. 8, HC Blue No. 9, HC Blue No. 10, HC Blue No. 11, HC Blue No. 12, HC Blue No. 13, HC Blue No. 14, HC Blue No. 15, HC Blue No.16, HC Blue No. 17, HC Blue No. 18, HC Brown No. 1, HC Brown No. 2, HC Green No. 1, HC Orange No. 1, HC Orange No. 2, HC Orange No. 3, HC Orange No. 5, HC Orange No. 6, HC Red No. 1, HC Red No. 3, HC Red No. 7, HC Red No. 8, HC Red No. 9, HC Red No. 10, HC Red No. 11, HC Red No. 13, HC Red No. 14, HC Red No. 15, HC Red No. 17, HC Red No. 18, HC Violet No. 1, HC Violet No. 2, HC Yellow No. 2, HC Yellow No. 4, HC Yellow No. 5, HC Yellow No. 6, HC Yellow No. 7, HC Yellow No. 8, HC Yellow No. 9, HC Yellow No. 10, HC Yellow No. 11, HC Yellow No. 12, HC Yellow No. 13, HC Yellow No. 14, HC Yellow No. 15, HC Yellow No. 16, HC Yellow No. 17, 2-Hydroxyethylamino-5-Nitroanisole, Hydroxyethyl-2-Nitro-p-Toluidine, 4-Hydroxypropylamino-3-Nitrophenol, 3-Methylamino-4-Nitrophenoxyethanol, 3-Nitro-4-Aminophenoxyethanol, 3-Nitro-p-Cresol, 2-Nitro-5-Glyceryl Methylaniline, 4-Nitroguaiacol, 3-Nitro-p-Hydroxyethylaminophenol, 2-Nitro-N-Hydroxyethyl-p-Anisidine, Nitrophenol, 4-Nitrophenyl Aminoethylurea, 4-Nitro-o-Phenylenediamine, 4-Nitro-m-Phenylenediamine, 4-Nitro-o-Phenylenediamine, 2-Nitro-p-Phenylenediamine, 6-Nitro-2,5-Pyridinediamine, 6-Nitro-o-Toluidine, Pigment Blue 15, Pigment Blue 15:1, Pigment Violet 23, Pigment Yellow 13, Solvent Black 3, Solvent Black 5, Solvent Blue 35, Solvent Yellow 85, Solvent Yellow 172, Tetrabromophenol Blue, Tetrahydro-6-Nitroquinoxaline.

The compositions according to the invention can contain, in addition to cysteine, reducing agents and antioxidants such as sodium sulphite, sodium metabisulphite, isoascorbic acid and ascorbic acid.

The compositions according to the invention can include sequestering agents or chelating agents.

Examples of sequestering agents derived from carboxylic acids comprise diethylenetriaminepentaacetic acid (DTPA), ethylenediamine-N,N'-disuccinic acid (EDDS) and trisodium ethylenediamenedisuccinate, ethylenediamenetetraacetic acid (EDTA) and the salts thereof such as disodium EDTA and tetrasodium EDTA, ethylenediamine-N,N'-diglutaric acid (EDDG), glycinamide-N,N'-disuccinic acid (GADS), 2-hydroxypropylenediamene-N,N'-disuccinic acid (HPDDS) and ethylenediamine-N,N' -bis(ortho-hydroxyphenylacetic acid) (EDDHA), N,N'-bis(2-hydroxybenzyl)ethylenediamine-N,N'-diacetic acid (HBED), nitrilotriacetic acid (NTA), methylglycinediacetic acid (MGDA), N-2-hydroxyethyl-N,N-diacetic acid and glyceryliminodiacetic acid, iminodiacetic-N-2-hydroxypropylsulphonic acid and N-(carboxymethyl) aspartic acid, 2-hydroxypropyl-3-sulphonic acid, beta-alanine-N,N'-diacetic acid, aspartic acid-N,N'-diacetic acid, iminodisuccinic acid (IDSA), ethanol diglycine acid, phosphonobutanetricarboxylic acid, N-dicarboxymethylglutamic acid and the salts thereof such as tetrasodium glutamate diacetate (GLDA) (Dissolvine GL38 or 45S ®).

Examples of chelating agents derived from mono- or polyphosphonic acid comprise diethylenetriaminepenta (methylenephosphonic) acid (DTPMP), ethane-1-hydroxy-1,1,2-triphosphonic acid (E1 HTP), ethane-2-hydroxy-1,1,2-triphosphonic acid (E2HTP), ethane-1,1,1-diphosphonic acid (EHDP), ethane-1,1,2-triphosphonic acid (ETP), ethylenediamene-tetramethylenephosphonic acid (EDTMP), hydroxyethane-1,1-diphosphonic acid (HEDP or etidronic acid) and salts such as disodium etidronate and tetrasodium etidronate.

Examples of chelating agents derived from polyphosphoric acid comprise sodium tripolyphosphate (STP), tetrasodium diphosphate, hexametaphosphoric acid, sodium metaphosphate and phytic acid.

The total content of sequestering agents preferably ranges from 0.01% to 8% by weight, more preferably from 0.05% to 5% by weight.

The compositions according to the invention can also contain one or more additives commonly used in the cosmetics industry, such as solvents, surfactants, emulsifiers, wetting agents, thickeners, conditioners, etc.

Examples of solvents include water, low-molecular-weight aliphatic mono- or polyalcohols, esters and ethers thereof, for example alkanols, in particular having 1 to 4 carbon atoms, such as ethanol, n-propanol, isopropanol, butanol and isobutanol; bivalent or trivalent alcohols, in particular having 2 to 6 carbon atoms, such as ethylene glycol, propylene glycol, 1,3-propanediol, 1,4-butanediol, 1,5-pentanediol, 1,6-hexanediol, 1,2,6-hexanetriol, glycerin, diethylene glycol, dipropylene glycol, polyalkylene glycols, such as triethylene glycol, polyethylene glycol, tripropylene glycol and polypropylene glycol; low-molecular-weight alkyl ethers of multivalent alcohols, such as ethylene glycol monomethyl ether, ethylene glycol monoethyl ether, ethylene glycol monopropyl ether or ethylene glycol monobutyl ether, diethylene glycol monomethyl ether or diethylene glycol monoethyl ether, triethylene glycol monomethyl ether or triethylene glycol monoethyl ether; ketones and keto alcohols, in particular having 3 to 7 carbon atoms, such as acetone, methyl ethyl ketone, diethyl ketone, methyl isobutyl ketone, methyl phenyl ketone, cyclopentanone, cyclohexanone and diacetone alcohol; ethers such as dibutyl ether, tetrahydrofuran, dioxane or diisopropylether; esters such as ethyl formate, methyl formate, methyl acetate, ethyl acetate, propyl acetate, butyl acetate, phenyl acetate, ethylene glycol monoethyl ether acetate or acetic acid hydroxyethyl ester; amides such as N-methylpyrrolidone; urea, tetramethyl urea and thiodiglycol.

The following can also be present: anionic, cationic, non-ionic, amphoteric or zwitterionic emulsifiers; wetting agents; surfactants, such as fatty alcohol sulphates, alkylsulphonates, alkylbenzene sulphonates, alkylmethyl ammonium salts, alkylbetaine, $\alpha$-olefin sulphonates, ethoxylated fatty alcohols, ethoxylated nonylphenols, fatty acid alkanolamines, ethoxylated esters of fatty acids, polyglycol ether sulphates of fatty acids and alkylpolyglycosides, thickeners, such as higher fatty alcohols, starches, cellulose derivatives, vaseline, paraffin oil, fatty acids and other fatty ingredients in emulsified form, water-soluble polymer thickeners, such as natural gums, guar gum, tara gum, xanthan gum, carob flour, pectin, dextran, agar-agar, amylose, amylopectin, dextrin, synthetic clays or hydrocolloids, such as polyvinyl alcohol; conditioning and restructuring agents such as lanolin derivatives, cholesterol, pantothenic acid, water-soluble cationic polymers, protein derivatives, amino acids, provitamins, vitamins, plant extracts, sugar and betaine; auxiliary agents such as electrolytes, antioxidants, fatty amides, sequestering agents, film-forming agents and preservatives, and beeswax.

In the compositions according to the invention, the addition of non-ionic and/or anionic surfactants, such as fatty alcohol sulphates, in particular lauryl sulphate and sodium cocoyl sulphate, can be particularly advantageous; ethoxylated fatty alcohol sulphates, in particular sodium lauryl ether sulphates with 2 to 4 molecular units of ethylene oxide, esters of ethoxylated fatty acids, ethoxylated nonylphenols, ethoxylated fatty alcohols, alkylbenzene sulphonates or fatty acid alkanolamides, in a total amount preferably ranging from about 0.1 to 30% by weight, more preferably 0.2 to 15% by weight.

Examples of cationic surfactants are quaternary ammonium compounds; ammonium halides such as alkyltrimethylammonium chlorides, dialkyldimethylammonium chlorides and trialkylmethylammonium chlorides. Specific examples are cetyltrimethylammonium chloride, stearyltrimethylammonium chloride, distearyldimethylammonium chloride, lauryldimethylammonium chloride, lauryldimethylbenzylammonium chloride, tricetylmethylammonium chloride and quaternised protein hydrolysates.

In addition to non-ionic organic thickeners with similar properties to wax and to non-ionic surfactants, the composition can include cationic resins such as Polyquaternium-6, Polyquaternium-7, Polyquaternium-10, Polyquaternium-11, Polyquaternium-22, Polyquaternium-35, Polyquaternium-37 and Polyquaternium-113, alone or mixed, in percentages ranging from about 0.1 to 6% by weight.

Optionally, the composition according to the invention can include hair-conditioning agents such as volatile or non-volatile silicone oils, natural and synthetic oils.

The triglycerides of plant or synthetic origin are preferably selected from liquid triglycerides of fatty acids comprising 6 to 30 carbon atoms, such as triglycerides of heptanoic or octanoic acid, sunflower oil, corn oil, soya oil, bone-marrow oil, grapeseed oil, sesame seed oil, hazelnut oil, apricot oil, macadamia oil, arara oil, castor oil, avocado oil, caprylic/capric acid triglycerides, jojoba oil and shea butter oil, and mixtures thereof. The composition can also comprise monosaccharides, oligosaccharides or polysaccharides.

Examples of suitable sugars include sucrose, glucose, galactose, ribose, fucose, maltose, fructose, mannose, arabinose, xylose and lactose and derivatives thereof, in particular alkyl derivatives, such as methyl derivatives, for example methyl glucose.

The total amount of said additives can range from about 0.1 to 6% by weight.

The compositions according to the invention are applied to the hair by the following methods:

1 - The composition is mixed with an oxidative solution immediately before colouring the hair, and a sufficient amount of ready-to-use dye mixture, generally about 60 to 200 grams, depending on the thickness and amount of the hair, is then applied to the hair.

The mixture is left on the hair for 5 to 60 minutes at a temperature ranging from 5 to 50°C, preferably for 35 minutes at 30°C; the hair is then rinsed with water and dried.

2 - The hair-colouring preparation is applied directly to the hair and left for 5 to 60 minutes at a temperature ranging from 5 to 50°C, preferably for 35 minutes at 30°C; the hair is then rinsed with water and dried. The hair-colouring preparation can be applied for several consecutive days until the desired colour depth is reached.

3 - The hair-colouring preparation is applied directly to the hair and then dried without rinsing. The hair-colouring preparation can be applied for several consecutive days until the desired depth is reached.

**DEFINITIONS**

The International Colour Chart (ICC) is a classification system for hair-colouring preparations according to which each dye corresponds to a code or number that defines the colour. In practice, the ICC system uses numbers to define the depth (level) and tone of a given colour. The colour "level" indicates how light or dark the shade is. The ICC (International Colour Chart) system uses numbers to define the depth of colour. The values range from 1 to 11, wherein 1 denotes the darkest shade (black) and 11 the lightest shade (platinum blonde).

Table A shows the usual names matching each number.

**Table A**

| Level | Level name |
|---|---|
| 1 | Black |
| 2 | Very dark brown |
| 3 | Dark brown |
| 4 | Medium brown |
| 5 | Light brown |
| 6 | Dark blonde |
| 7 | Medium blonde |
| 8 | Light blonde |
| 9 | Very light blonde |
| 10 | Lightest blonde |
| 11 | Platinum blonde |

The tone indicates how cool or warm a colour is (gold, ash or copper). Although the level measurement is almost identical, each manufacturer follows different criteria when classifying tone, which is indicated by a number, usually placed after the level, separated by a decimal point ".", a comma "," or a slash "/". The classification followed by the Applicant is set out in Table B below:

**Table B**

| Tone number | Tone name |
|---|---|
| 0 | Natural (grey-neutral) |
| 1 | Ash (blue) |
| 2 | Irisé (violet) |
| 3 | Gold (yellow) |
| 4 | Copper (orange) |
| 5 | Mahogany (red+violet) |
| 6 | Red (red) |
| 7 | Khaki (green) |
| 8 | Pearl |

Some hair-colouring preparations can have a double tone, and it is usual to place two numbers after the decimal point of the level to express said characteristic. For example, in the number 7.21 in the colour chart, the first number indicates the medium blonde level (7), the second indicates the irisé tone (2), and the third indicates a secondary ash tone (1), thus defining the colour "medium irisé ash blonde". Some hair-colouring preparations do not have a level, only a tone; said preparations are called "toners", and indicated by a code such as type .2 (violet toner).

**EXAMPLES**

The ingredients listed in the examples are identified by the INCI nomenclature, as defined above.

Table 1 shows the compositions exemplified in gel form. In the examples and the description, the percentages are always expressed by weight, unless otherwise indicated.

Formula F1 represents a violet toner, namely a dye with no level and with tone 2, without resorcinol or derivatives thereof. It is a formula characterised by the presence of MAPs and an antioxidant-chelating system based on isoascorbic acid, sodium sulphite and EDTA. Formulas F2 to F12 represent formula F1 with the addition of a different antioxidant, selected from those most commonly used on the market or specified in the literature.

Table 1 - Gel toners without resorcinol and derivatives

| INGREDIENTS (INCI) | F1 | F2 | F3 | F4 | F5 | F6 | F7 | F8 | F9 | F10 | F11 | F12 |
|---|---|---|---|---|---|---|---|---|---|---|---|---|
| AQUA (WATER) | Q.s. to 100 | Q.s. to 100 | Q.s. to 100 | Q.s. to 100 | Q.s. to 100 | Q.s. to 100 | Q.s. to 100 | Q.s. to 100 | Q.s. to 100 | Q.s. to 100 | Q.s. to 100 | Q.s. to 100 |
| PROPYLENE GLYCOL | 3.5 | 3.5 | 3.5 | 3.5 | 3.5 | 3.5 | 3.5 | 3.5 | 3.5 | 3.5 | 3.5 | 3.5 |
| ETHOXYDIGLYCOL | 3.0 | 3.0 | 3.0 | 3.0 | 3.0 | 3.0 | 3.0 | 3.0 | 3.0 | 3.0 | 3.0 | 3.0 |
| HYDROXYETHYLCELLULOSE | 2.4 | 2.4 | 2.4 | 2.4 | 2.4 | 2.4 | 2.4 | 2.4 | 2.4 | 2.4 | 2.4 | 2.4 |
| ISOPROPANOLAMINE | 1.6 | 1.6 | 1.6 | 1.6 | 1.6 | 1.6 | 1.6 | 1.6 | 1.6 | 1.6 | 1.6 | 1.6 |
| SODIUM POLYACRYLATE | 1 | 1 | 1 | 1 | 1 | 1 | 1 | 1 | 1 | 1 | 1 | 1 |
| PEG-40-HYDROGENATED CASTOR OIL | 0.5 | 0.5 | 0.5 | 0.5 | 0.5 | 0.5 | 0.5 | 0.5 | 0.5 | 0.5 | 0.5 | 0.5 |
| SODIUM SULPHITE | 0.4 | 0.4 | 0.4 | 0.4 | 0.4 | 0.4 | 0.4 | 0.4 | 0.4 | 0.4 | 0.4 | 0.4 |
| ERYTHORBIC ACID | 0.3 | 0.3 | 0.3 | 0.3 | 0.3 | 0.3 | 0.3 | 0.3 | 0.3 | 0.3 | 0.3 | 0.3 |
| TETRASODIUM EDTA | 0.26 | 0.26 | 0.26 | 0.26 | 0.26 | 0.26 | 0.26 | 0.26 | 0.26 | 0.26 | 0.26 | 0.26 |
| PARFUM (FRAGRANCE) | 0.2 | 0.2 | 0.2 | 0.2 | 0.2 | 0.2 | 0.2 | 0.2 | 0.2 | 0.2 | 0.2 | 0.2 |
| POLYQUATERNIUM-7 | 0.15 | 0.15 | 0.15 | 0.15 | 0.15 | 0.15 | 0.15 | 0.15 | 0.15 | 0.15 | 0.15 | 0.15 |
| TOLUENE-2,5-DIAMINE SULPHATE (PTD) | 0.128 | 0.128 | 0.128 | 0.128 | 0.128 | 0.128 | 0.128 | 0.128 | 0.128 | 0.128 | 0.128 | 0.128 |
| 2-METHYL-5-HYDROXYETHYLAMINOPHENO L (PAOX) | 0.065 | 0.065 | 0.065 | 0.065 | 0.065 | 0.065 | 0.065 | 0.065 | 0.065 | 0.065 | 0.065 | 0.065 |
| m-AMINOPHENOL (MAP) | 0.0138 | 0.0138 | 0.0138 | 0.0138 | 0.0138 | 0.0138 | 0.0138 | 0.0138 | 0.0138 | 0.0138 | 0.0138 | 0.0138 |
| HYDROXYETHYL-3,4-METHYLENEDIOXY ANILINE HCL (HMOC) | 0.015 | 0.015 | 0.015 | 0.015 | 0.015 | 0.015 | 0.015 | 0.015 | 0.015 | 0.015 | 0.015 | 0.015 |
| CARBOMER | 0.01 | 0.01 | 0.01 | 0.01 | 0.01 | 0.01 | 0.01 | 0.01 | 0.01 | 0.01 | 0.01 | 0.01 |
| PHENYL METHYL PYRAZOLONE (PMP) | - | 0.1 | 0.2 | - | - | - | - | - | - | - | - | - |
| TBHQ (t-Butyl Hydroquinone) | - | - | - | 0.1 | - | - | - | - | - | - | - | - |
| PYROGALLOL | - | - | - | - | 0.1 | - | - | - | - | - | - | - |
| THIOLACTIC ACID | - | - | - | - | - | 0.1 | - | - | - | - | - | - |
| THIOGLYCERIN | - | - | - | - | - | - | 0.1 | - | 0.2 | - | - | - |

(continued)

| INGREDIENTS (INCl) | F1 | F2 | F3 | F4 | F5 | F6 | F7 | F8 | F9 | F10 | F11 | F12 |
|---|---|---|---|---|---|---|---|---|---|---|---|---|
| D,L- CYSTEINE HCL | - | - | - | - | - | - | - | 0.1 | - | 0.2 | 0.3 | 0.4 |

Table 2 shows the cream compositions in deep red shade (6.66I) used in the examples below. In particular, formulas F16* and F17* are formulas according to the invention, while F13 is the reference standard (STD deep red).

**Table 2 - Hair-colouring creams in shade 6.66I, without resorcinol and derivatives**

| | F13 (STD deep red) | F14 | F15 | F16* | F17* | F18 |
|---|---|---|---|---|---|---|
| **INGREDIENTS (INCI)** | **w/w%** | **w/w%** | **w/w%** | **w/w%** | **w/w%** | **w/w%** |
| AQUA (WATER) | Q.s. to 100 | Q.s. to 100 | Q.s. to 100 | Q.s. to 100 | Q.s. to 100 | Q.s. to 100 |
| CETEARYL ALCOHOL | 8 | 8 | 8 | 8 | 8 | 8 |
| CETEARETH-50 | 7 | 7 | 7 | 7 | 7 | 7 |
| STEARYL ALCOHOL | 6 | 6 | 6 | 6 | 6 | 6 |
| PROPYLENE GLYCOL | 5 | 5 | 5 | 5 | 5 | 5 |
| PEG-40-HYDROGENATED CASTOR OIL | 3 | 3 | 3 | 3 | 3 | 3 |
| LAURYL ALCOHOL | 3 | 3 | 3 | 3 | 3 | 3 |
| 1-HYDROXYETHYL 4,5-DIAMINO PYRAZOLE SULPHATE | 2.47 | 2.47 | 2.47 | 2.47 | 2.47 | 2.47 |
| AMMONIA | 1.8 | 1.8 | 1.8 | 1.8 | 1.8 | 1.8 |
| ETHANOLAMINE | 1.5 | 1.5 | 1.5 | 1.5 | 1.5 | 1.5 |
| COCAMIDOPROPYL BETAINE | 1.2 | 1.2 | 1.2 | 1.2 | 1.2 | 1.2 |
| MYRISTYL ALCOHOL | 1.2 | 1.2 | 1.2 | 1.2 | 1.2 | 1.2 |
| m-AMINOPHENOL (MAP) | 1 | 1 | 1 | 1 | 1 | 1 |
| DIETHYLHEXYL AZELATE | - | - | 1 | 1 | - | - |
| DIOCTYLDODECYL AZELATE | - | - | - | - | 1 | 1 |
| PARFUM (FRAGRANCE) | 0.8 | 0.8 | 0.8 | 0.8 | 0.8 | 0.8 |
| DECYLTETRADECANOL | 0.7 | 0.7 | 0.7 | 0.7 | 0.7 | 0.7 |
| D, L CYSTEINE HCL | - | 0.4 | - | 0.4 | 0.4 | - |
| ERYTHORBIC ACID | 0.4 | 0.4 | 0.4 | 0.4 | 0.4 | 0.4 |
| SODIUM SULPHITE | 0.4 | 0.4 | 0.4 | 0.4 | 0.4 | 0.4 |
| ACID RED 92 (RED 28) | 0.38 | 0.38 | 0.38 | 0.38 | 0.38 | 0.38 |
| TETRASODIUM EDTA | 0.26 | 0.26 | 0.26 | 0.26 | 0.26 | 0.26 |
| 4-AMINO-2-HYDROXYTOLUENE (PAOC) | 0.2 | 0.2 | 0.2 | 0.2 | 0.2 | 0.2 |
| p-AMINOPHENOL (PAP) | 0.05 | 0.05 | 0.05 | 0.05 | 0.05 | 0.05 |
| HC YELLOW NO. 2 | 0.038 | 0.038 | 0.038 | 0.038 | 0.038 | 0.038 |

Table 3 shows the cream compositions in platinum blonde irisé ash shade (11.21) used in the examples below. In particular, formulas F20* and F21* are formulas according to the invention, while F19 is the reference standard (STD platinum).

**Table 3 - Hair-colouring creams in shade 11.21 without resorcinol and derivatives**

|  | F19 (STD platinum) | F20* | F21* |
|---|---|---|---|
| **INGREDIENTS (INCI)** | **w/w%** | **w/w%** | **w/w%** |
| AQUA (WATER) | Q.s. to 100 | Q.s. to 100 | Q.s. to 100 |
| CETYL ALCOHOL | 15 | 15 | 15 |
| DECYLTETRADECANOL | 7 | 7 | 7 |
| PROPYLENE GLYCOL | 6 | 6 | 6 |
| OLEYL ALCOHOL | 5 | 5 | 5 |
| CETEARETH-50 | 4 | 4 | 4 |
| AMMONIA | 4 | 4 | 4 |
| AMMONIUM LAURYL SULPHATE | 3.8 | 3.8 | 3.8 |
| STEARIC ACID | 1.5 | 1.5 | 1.5 |
| ETHANOLAMINE | 1.5 | 1.5 | 1.5 |
| PALMITIC ACID | 1.2 | 1.2 | 1.2 |
| PEG-15 COCOPOLYAMINE | 1 | 1 | 1 |
| DIETHYLHEXYL AZELATE | - | 2 | - |
| DIOCTYLDODECYL AZELATE | - | - | 2 |
| TETRASODIUM EDTA | 0.9 | 0.9 | 0.9 |
| BISABOLOL | 0.8 | 0.8 | 0.8 |
| PARFUM | 0.8 | 0.8 | 0.8 |
| TOCOPHEROL | 0.5 | 0.5 | 0.5 |
| L-CYSTEINE HCL | - | 1 | 1 |
| SODIUM SULPHITE | 0.4 | 0.4 | 0.4 |
| ERYTHORBIC ACID | 0.3 | 0.3 | 0.3 |
| POLYQUATERNIUM-22 | 0.2 | 0.2 | 0.2 |
| m-AMINOPHENOL (MAP) | 0.058 | 0.058 | 0.058 |
| TOLUENE-2,5-DIAMINE SULPHATE (PTD) | 0.0125 | 0.0125 | 0.0125 |

Table 4 shows examples of liquid toners according to the invention.

**Table 4 - Violet liquid toners with resorcinol derivatives**

|  | F22* | F23* |
|---|---|---|
| **INGREDIENTS (INCI)** | **w/w%** | **w/w%** |
| AQUA (WATER) | Q.s. to 100 | Q.s. to 100 |
| UNDECETH-3 | 17 | 17 |
| OLEIC ACID | 13 | 13 |
| ISOPROPYL ALCOHOL | 12 | 12 |
| PROPYLENE GLYCOL | 9 | 9 |
| LAURETH-2 | 4 | 4 |
| ARGININE | 3 | 3 |
| POTASSIUM HYDROXIDE | 3 | 3 |
| PEG-15 COCOPOLYAMINE | 1.5 | 1.5 |

(continued)

| INGREDIENTS (INCI) | F22* w/w% | F23* w/w% |
|---|---|---|
| PEG-90 GLYCERYL ISOSTEARATE | 0.5 | 0.5 |
| CETRIMONIUM CHLORIDE | 0.5 | 0.5 |
| ERYTHORBIC ACID | 0.4 | 0.4 |
| SODIUM SULPHITE | 0.4 | 0.4 |
| L-CYSTEINE HCL | 0.3 | 0.3 |
| EDTA | 0.3 | 0.3 |
| DIOCTYLDODECYL AZELATE | - | 0.05 |
| m-AMINOPHENOL (MAP) | 0.089 | 0.089 |
| p-AMINOPHENOL | 0.08 | 0.08 |
| 4-AMINO-2-HYDROXYTOLUENE | 0.064 | 0.064 |
| p-PHENYLENEDIAMINE (PPD) | 0.064 | 0.064 |
| 2-METHYL RESORCINOL | 0.02 | 0.02 |
| DIETHYLHEXYL AZELATE | 0.05 | - |

## PRELIMINARY TEST OF MAINTENANCE OF VIOLET TONE: PANEL TEST

The test evaluates the efficacy of antioxidants in preserving the violet tone during storage, and preserving the appearance of the product. The test was conducted on locks of natural lightest blonde hair (level 10) and yak hair.

The compositions according to Tables 1 and 2, mixed in a 1:2 ratio with Yellow Peroxide 5 vol activator from the Alfaparf Group, were applied to the locks for 10 minutes at 30°C, then rinsed off and dried. A group of 5 expert panelists evaluated the following parameters:

1) Colour of gel just squeezed from tube: ideally colourless or pale yellow
2) Colour of locks after application of hair-colouring composition:

violet tone present = 3
partial violet tone = 2
weak violet tone = 1
no violet tone = 0

3) The evaluation was conducted at time zero (dye just formulated in the laboratory) and after 15 days' storage at room temperature (25°C) and in a stove at 50°C.

The results are shown in Table 5.

**Table 5**

| COMPOSITION | Gel colour at T0 | Gel colour after 15 days at RT | Gel colour after 15 days at 50°C | Violet tone of locks at T0 | Violet tone of locks after 15 days at RT | Violet tone of locks after 15 days at 50°C |
|---|---|---|---|---|---|---|
| F1 | yellow | orange | orange | 3 | 1 | 0 |
| F2 (PHENYL METHYL PYRAZOLONE 0.1%) | yellow | orange | orange | 3 | 1 | 1 |

(continued)

| COMPOSITION | Gel colour at T0 | Gel colour after 15 days at RT | Gel colour after 15 days at 50°C | Violet tone of locks at T0 | Violet tone of locks after 15 days at RT | Violet tone of locks after 15 days at 50°C |
|---|---|---|---|---|---|---|
| F3 (PHENYL METHYL PYRA-ZOLONE 0.2%) | yellow | orange | orange | 0 | - | - |
| F4 (TBHQ 0.1%) | Yellowish-orange | Yellowish-orange | Yellowish-orange | 3 | 1 | 0 |
| F5 (PYROGALLOL 0.1%) | yellow | Greenish-brown | Greenish-brown | 3 | 0 | 0 |
| F6 (THIOLACTIC ACID 0.1%) | Pale yellow | Yellowish-green | Yellowish-green | 3 | 0 | 0 |
| F7 (THIOGLYCERIN 0.1%) | Pale yellow | Yellow | Yellow | 3 | 1 | 0 |
| F8 (CYSTEINE 0.1%) | yellow | Yellowish-orange | Yellowish-orange | 3 | 1 | 0 |
| F9 (THIOGLYCERIN 0.2%) | Pale yellow | Yellowish-orange | Yellow | 3 | 1 | 0 |
| F10 (CYSTEINE 0.2%) | Pale yellow | Pale yellow | Pale yellow | 3 | 1 | 1 |
| F11 (CYSTEINE 0.3%) | colourless | Pale yellow | Pale yellow | 3 | 3 | 3 |
| F12 (CYSTEINE 0.4%) | colourless | colourless | colourless | 3 | 3 | 3 |

The results shown in Table 5 clearly demonstrate that pyrogallol, thiolactic acid and TBHQ, even at 0.1%, worsen the appearance of the gel compared with reference formula F1, without improving the violet tone.

0.1% PMP does not improve the violet tone, but slightly improves the appearance of the gel. However, at the concentration of 0.2%, PMP alters the colour result, with total loss of violet tone already at time zero.

0.1% thioglycerin does not improve the violet tone, but considerably improves the appearance of the gel, and is better than 0.1% cysteine. However, if the percentage is increased to 0.2%, cysteine performs better than 0.2% thioglycerin.

Formulas F11 and F12 (containing 0.3% and 0.4% cysteine) are the best in terms of stability, as regards both the colour of the gel and maintenance of violet tone.

**PRELIMINARY TEST OF MAP STABILITY: HPLC**

The test was conducted with an Agilent 1200 HPLC instrument with a Raptor arc-18 column and elution gradient with acetonitrile and buffer at pH 6.

The formulas shown in Tables 1 and 2 were designed to establish the loss of MAP and PAOX over time. The results shown in Table 6 are expressed in terms of % loss after 15 days at RT and 15 days at 50°C vs T0.

Table 6

| COMPOSITION | % MAP loss after 15 days at RT | % MAP loss after 15 days at 50°C | % PAOX loss after 15 days at RT | % PAOX loss after 15 days at 50°C |
|---|---|---|---|---|
| F1 | -71 | -66 | -49 | -50 |
| F2 (PHENYL METHYL PYRAZOLONE 0.1%) | -39 | -45 | -21 | -39 |
| F4 (TBHQ 0.1%) | -38 | -65 | -43 | -52 |

(continued)

| COMPOSITION | % MAP loss after 15 days at RT | % MAP loss after 15 days at 50°C | % PAOX loss after 15 days at RT | % PAOX loss after 15 days at 50°C |
|---|---|---|---|---|
| F5 (PYROGALLOL 0.1%) | -19 | -26 | -50 | -56 |
| F6 (THIOLACTIC ACID 0.1%) | -37 | -68 | -47 | -49 |
| F7 (THIOGLYCERIN 0.1%) | -39 | -57 | -34 | -58 |
| F8 (CYSTEINE 0.1%) | -24 | -18 | -54 | -65 |
| F9 (THIOGLYCERIN 0.2%) | -4 | 0 | -51 | -60 |
| F10 (CYSTEINE 0.2%) | -10 | -2 | -18 | -27 |
| F11 (CYSTEINE 0.3%) | 0 | 0 | -7 | -13 |
| F12 (CYSTEINE 0.4%) | 0 | 0 | 0 | -3 |

As shown in Table 6, 0.1% thiolactic acid and TBHQ only improve MAP degradation at time zero, but do not preserve MAP and PAOX over time.

0.1% pyrogallol partly prevents MAP degradation, but is unable to improve PAOX degradation.

0.1% PMP stabilises both MAP and PAOX, but cannot be used at higher percentages because it alters the colour result, as demonstrated in the preceding example.

0.2% thioglycerin only improves MAP degradation at both RT and 50°C, but does not improve PAOX degradation in the slightest, whereas 0.2% cysteine improves both MAP and PAOX degradation.

Cysteine at concentrations ranging from 0.3% to 0.4% considerably improves both MAP and PAOX degradation. In particular, formula F12 preserves the two dyes unchanged.

## FINAL MAP STABILITY TEST: HPLC

Loss of MAP over time in the formulas shown in Table 3 was measured with HPLC. The results, described in Table 7, are expressed in terms of % loss after 15 days at RT and 6 months at RT vs T0.

**Table 7**

| COMPOSITION | % MAP loss after 15 days at RT | % MAP loss after 6 months at RT |
|---|---|---|
| F18 | -66 | -85 |
| F19* | -12 | -18 |
| F20* | -9 | -16 |

The data presented in Table 7 confirm the efficacy of cysteine in stabilising MAP, and the presence of azelaic acid esters has no adverse effect on this stabilization.

## COLORIMETRIC TEST: HEIGHT OF TONE (LEVEL)

Antioxidants not only stabilise dyes over time, but can also adversely affect the colour development reaction, producing a different result in terms of height of tone (level) and colour. The impact of cysteine on the colour result was therefore evaluated using permanent colouring preparation formulas in cream form with ammonia in shade 6.66 I (deep red), because red is the colour most liable to fade. It was decided to work with the deep red 6.66I colour formulas shown in Table 3, which allow easy evaluation of variations in height of tone (level). The test was performed on IHIP level 5 natural locks of hair with 5% grey hair, which were dyed with formulas F13, F14, F15, F16*, F17* and F18 at the ratio of 1:1.5 with Alfaparf Oxid'o 20 vol. activator. After being left for 15 minutes at 30°C, the locks were rinsed and dried. A Konica Minolta colorimeter was used to evaluate the colour.

In the CIELAB colour space, L* indices height of tone or level L*, ranging from 0 (black) to 100 (white), while a* and b* are the colour coordinates indicating the colour directions: +a* is the direction of red, -a* is the direction of green, +b* is the direction of yellow and -b* is the direction of blue. High values of parameter L* correspond to light colours, while low values indicate darker colours. Table 8 shows the values of L* and $\Delta$ L* for the formulas analysed.

**Table 8**

| COMPOSITION | L* | ΔL* %vsF12 |
|---|---|---|
| F13 (standard deep red) | 22.87 | - |
| F14 | 23.51 | +2.8% |
| F15 | 22.64 | -1% |
| F16* | 22.95 | +0.35% |
| F17* | 22.93 | + 0.26% |
| F18 | 22.45 | -1.8% |

The values set out in Table 8 demonstrate that formula F14 only containing cysteine tends to lighten the colour (while maintaining the red tone), and the one only containing diethylhexyl azelate tends to darken it, whereas the combination of the two ingredients (formula F16*) maintains the colour result at an optimum level. The same beneficial effect is obtained by combining cysteine with dioctyldodecyl azelate (formula F17* vs F14 which lightens and F18 which darkens the colour).

**COLORIMETRIC TEST: COLOUR DIFFERENCE**

Bleaching makes the hair more porous, thus preventing the ideal colour result from being obtained. Evaluating oxidative dyes on bleached hair thus makes it easier to amplify and appreciate the differences in results between different formulations. Once again, it was decided to work with the deep red 6.66I colour formulas shown in Table 3, which are the easiest to evaluate.
The test was performed on IHIP level 10 natural locks of hair bleached with Alfaparf BB bleach 7 levels bleaching powder mixed at the ratio of 1:2 with Alfaparf Oxid'O 40 vol.. After being left for 40 minutes at 30°C, the locks were rinsed and dried.
The locks were dyed with formulas F13, F14, F15, F16*, F17 and F18 at the ratio of 1:1.5 with Alfaparf Oxid'O 20 volumes activator. After being left for 35 minutes at 30°C, the locks were rinsed and dried. A Konica Minolta colorimeter was used to evaluate the colour. Differences in colour can be expressed by the ΔE values, which are defined by the equation:

$$\Delta E = [(\Delta L^*)2 + (\Delta a^*)2 + (\Delta b^*)2]1/2$$

ΔE< 1 indicates that two colours are substantially equal
Table 9 shows the values of E and ΔE for the formulas analysed

**Table 9**

| COMPOSITION | E | ΔE vs F12 |
|---|---|---|
| F13 (standard deep red) | 33.64 | - |
| F14 | 32.40 | -1.24 |
| F15 | 34.11 | +0.47 |
| F16* | 33.23 | -0.41 |
| F17* | 33. 22 | -0.42 |
| F18 | 34.18 | +0.54 |

The values set out in Table 9 demonstrate that formula F14 only containing cysteine tends to give a different colour result from standard formula F13. Formulas F15 and F18 containing azelaic acid esters without cysteine exhibit no significant colour differences from standard formula F13. Combining diethylazelate or dioctyldodecyl azelate with cysteine in formulas F16* and F17* eliminates the adverse effect of cysteine, optimally preserving the colour result.

**TENSILE PROPERTY TEST**

A DIA-STRON MTT670 dynamometer was used to evaluate the tensile properties of the hair. The evaluation was conducted on suitably damaged locks of hair in order to evaluate more effectively any beneficial/reparatory effect of the oxidative dye formulations. The natural IHIP 90% grey locks were damaged with a mild bleach and a mild permanent dye to simulate the average damage to hair that undergoes chemical treatments. The method is described in detail below.

Wash the locks with standard shampoo (SLS + cocoamidopropylbetaine sol.).

Mix Alfaparf BB bleach Level 7 bleaching powder with Alfaparf Oxid'O 20 Vol. at the ratio of 1:2, and dilute with demineralised water at the ratio of 1:2 (pH 9.5). Distribute the product on the locks and leave to act for 30 minutes at room temperature. Rinse under running water. Dilute ammonium thioglycolate with demineralised water at the ratio of 1:10, adjust to pH 9.0 with ammonia, and immerse the locks for 30 minutes.

Rinse the locks under running water, then immerse them in a 35% hydrogen peroxide solution diluted with demineralised water at the ratio of 1:15. After 10 minutes, rinse and dry. The locks thus damaged are dyed with formulas F13, F14, F15, F16* and F17* at the ratio of 1:1.5 with Alfaparf Oxid'O 20 volumes activator. After being left for 35 minutes at 30°C, the locks were rinsed and dried. The work required to break hair fibres subjected to the 4 treatments is measured with a dynamometer. The more work required, the greater the tensile strength of the hair. The values are set out in Table 10.

**Table 10**

| COMPOSITION | Total average work (Joules/m3) | Δ % Work vs damaged undyed locks |
|---|---|---|
| No hair-colouring preparation | 31011111 | - |
| F13 (STD deep red) | 22658666 | -27% |
| F14 | 2160000 | -30% |
| F15 | 31814000 | +2.6% |
| F16* | 36791000 | +19% |
| F17* | 36802000 | +19% |
| F18 | 31784000 | +2,5% |

The results set out in Table 10 demonstrate that Standard dye F13 worsens the tensile strength of the hair, the addition of cysteine does not affect the total work, the addition of diethylhexyl azelate improves the tensile strength of the hair, but the improvement is amplified by combining cysteine with diethylhexyl azelate. Formulas F16* and F17* according to the invention have a repairing effect on damaged hair.

**SHINE TEST**

The "shine" parameter, an effect determined by the reflection of light on the hair, is measurable with the SAMBA Hair System® instrument (Bossanova Technologies). Differences in shine were evaluated with the Reich-Robbins method (Light scattering and shine measurements of human hair: A sensitive probe of the hair surface. J.Soc. Cosmet. Chem., 44, 221-234. July/August 1993).

The measurement was conducted on the same locks as in the preceding example. The results are set out in Table 11.

**Table 11**

| COMPOSITION | Average shine (Guiolet) | Δ % shine compared with standard dye |
|---|---|---|
| F13 (STD) | 464.8425 | - |
| F14 | 461.0725 | -0.8% |
| F15 | 472.23 | +1.6% |
| F16* | 506.355 | +8.9% |
| F17* | 515.562 | +11% |
| F18 | 470.25 | +1.2% |

The values set out in Table 11 demonstrate that the combination of cysteine and diethylhexyl azelate in formula F15* according to the invention gives rise to an increase in the shine of the hair compared with the standard formula. The two ingredients individually do not produce the same result.

**Claims**

1. Permanent hair-colouring compositions, comprising aminophenol oxidative dyes and at least one alkaliser, and **characterised by** a combination of cysteine with azelaic acid esters selected from diethylhexyl azelate and dioctyldodecyl azelate.

2. Compositions according to claim 1 wherein cysteine is in D, L or racemic form.

3. Compositions according to claims 1 and 2 wherein resorcinol is not present.

4. Compositions according to claims 1 to 3 comprising a direct dye.

5. Compositions according to claims 1 to 4 wherein the alkaliser is selected from ammonia, monoethanolamine, 1-amino-2-propanol, 2-amino-2-methyl-propanol (AMP), 2-amino-2-methyl-1,3-propanediol, 2-amino-2-ethyl-1,3-propanediol and tris(hydroxymethyl)-aminomethane, sodium hydroxide, potassium hydroxide, urea, allantoin, arginine, tripotassium phosphate, sodium saccharin, triethanolamine, dimethylglucamine, calcium sodium phosphosilicate, basic amino acids or combinations thereof.

6. Compositions according to any one of claims 1 to 5 in ready-to-use form, optionally in a mixture with an activator.

7. Compositions according to claim 6 wherein the activator is hydrogen peroxide.

8. Compositions according to any one of claims 1 to 7 wherein the concentration by weight of cysteine ranges between 0.3 and 1%.

9. Compositions according to any one of claims 1 to 8 wherein the concentration by weight of azelaic acid esters ranges between 0.05 and 2%.

# EUROPEAN SEARCH REPORT

**Application Number**

EP 24 20 9423

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2023/037788 A1 (SHI MINLI [US] ET AL) 9 February 2023 (2023-02-09) <br> * paragraph [0018]; claims 65, 66, 74 * <br> * paragraph [0213]; examples 4, 5, 6 * <br> - - - - - | 1-9 | INV. <br> A61K8/19 <br> A61K8/37 <br> A61K8/41 <br> A61K8/44 <br> A61Q5/10 <br> A61Q5/00 |
| A | JP 2005 213232 A (POLA CHEM IND INC) 11 August 2005 (2005-08-11) <br> * paragraphs [0002], [0004], [0009]; claims 1, 5 * <br> * examples 1-3 * <br> - - - - - | 1-9 | |
| A | US 5 653 968 A (CARBALLADA JOSE ANTONIO [US] ET AL) 5 August 1997 (1997-08-05) <br> * column 1, lines 1-4 * <br> * column 22, lines 34-55 * <br> * column 23, lines 14-22 * <br> - - - - - | 1-9 | |
| A | US 2018/161258 A1 (HINDLEY MICHAEL CHRISTOPHER [GB]) 14 June 2018 (2018-06-14) <br> * example 3 * <br> - - - - - | 1-9 | TECHNICAL FIELDS SEARCHED (IPC) |
| A | Temix Oleo: "Temest Z110: DiOtyldodecyl azelate", , 1 March 2021 (2021-03-01), XP093149086, Retrieved from the Internet: URL:https://digital.teknoscienze.com/digit al/beauty_horizons_4_2021_ita/scheda_ingre diente_temest_z110/231651/Temest_Z110_summ ary.pdf [retrieved on 2024-04-08] * the whole document * <br> - - - - - | 1-9 | A61K <br> A61Q |

-/--

The present search report has been drawn up for all claims

| Place of search | Date of completion of the search | Examiner |
|---|---|---|
| Munich | 12 February 2025 | Grenouillat, N |

page 1 of 2

Europäisches
Patentamt

European
Patent Office

Office européen
des brevets

# EUROPEAN SEARCH REPORT

Application Number

EP 24 20 9423

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Temix Oleo: "Temest J110: Diethylhexyl Azelate", , 1 March 2021 (2021-03-01), XP093149089, Retrieved from the Internet: URL:https://static1.squarespace.com/static /5c3512d19d5abbb682808ca2/t/61936a04028c17 5b4132d939/1637050885130/Temest+J110.pdf [retrieved on 2024-04-08] * the whole document * | 1-9 | |

- - - - -

TECHNICAL FIELDS
SEARCHED (IPC)

The present search report has been drawn up for all claims

| Place of search | Date of completion of the search | Examiner |
|---|---|---|
| Munich | 12 February 2025 | Grenouillat, N |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 20 9423

This annex lists the patent family members relating to the patent documents cited in the above-mentioned European search report.
The members are as contained in the European Patent Office EDP file on
The European Patent Office is in no way liable for these particulars which are merely given for the purpose of information.

12-02-2025

| Patent document cited in search report | Publication date | Patent family member(s) | | Publication date |
|---|---|---|---|---|
| US 2023037788 A1 | 09-02-2023 | NONE | | |
| JP 2005213232 A | 11-08-2005 | JP | 4205602 B2 | 07-01-2009 |
| | | JP | 2005213232 A | 11-08-2005 |
| US 5653968 A | 05-08-1997 | CN | 1219122 A | 09-06-1999 |
| | | EP | 0906080 A1 | 07-04-1999 |
| | | JP | H11506137 A | 02-06-1999 |
| | | US | 5653968 A | 05-08-1997 |
| | | WO | 9734573 A1 | 25-09-1997 |
| US 2018161258 A1 | 14-06-2018 | BR | 112017025043 A2 | 07-08-2018 |
| | | CN | 107645949 A | 30-01-2018 |
| | | EP | 3302413 A1 | 11-04-2018 |
| | | ES | 2922810 T3 | 20-09-2022 |
| | | JP | 6737815 B2 | 12-08-2020 |
| | | JP | 2018515577 A | 14-06-2018 |
| | | KR | 20180011771 A | 02-02-2018 |
| | | PL | 3302413 T3 | 21-11-2022 |
| | | US | 2018161258 A1 | 14-06-2018 |
| | | WO | 2016189276 A1 | 01-12-2016 |
| | | ZA | 201707527 B | 28-11-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20160214925 A
- WO 2007072892 A
- JP 2006288613 B
- US 11517514 B
- WO 2023163903 A
- US 11285091 B
- US 4875902 A
- JP 4995441 B
- US 20090265865 A
- US 7753966 B

### Non-patent literature cited in the description

- Recent Advancements in Natural Plant Colorants Used for Hair Dye Applications: A Review. *Molecules*, November 2022, vol. 27 (22), 8062
- Light scattering and shine measurements of human hair: A sensitive probe of the hair surface. *J.Soc. Cosmet. Chem*, July 1993, vol. 44, 221-234